# EUROPEAN PATENT APPLICATION

(11) **EP 3 326 675 A1**
(43) Date of publication of application: **30.05.2018**
(21) Application number: 16830181.0
(22) Date of filing: 10.06.2016
(51) Int. Cl.: A61M 5/32, A61J 1/05, A61M 5/178

(54) **NEEDLE UNIT**

(30) Priority: 24.07.2015 JP 2015147250
(71) Applicant: Otsuka Pharmaceutical Factory, Inc., Tokushima 772-8601 (JP)
(72) Inventor: NISHIOKA, Masaki, Naka-gun Tokushima 771-5209 (JP); TAKIGUCHI, Osamu, Naka-gun Tokushima 771-5209 (JP); HAMAI, Katsuyoshi, Naka-gun Tokushima 711-5209 (JP); MASUDA, Tetsuya, Naka-gun Tokushima 771-5209 (JP); INOUE, Fujio, Naruto-shi Tokushima 772-8601 (JP); TAKEDA,Koichi, Naruto-shi Tokushima 772-8601 (JP)
(74) Representative: Webster, Jeremy Mark
(86) International application number: PCT/JP2016/067458
(87) International publication number: WO 2017/018076

(57) **Abstract**

The present invention provides a needle unit configured to be mounted to medical syringe, the needle unit including a needle member having a longitudinally extending inner space and having a needle hole formed in a side surface of a front end and an opening formed in a base end, the inner space being in communication with the outside through the needle hole and the opening, a base portion for supporting the base end of the needle member and communicating between the syringe and the inner space of the needle member by being attached to the syringe, a slide member movable along an outer peripheral surface of the needle member and capable of taking a first position to close the needle hole and a second position to uncover the needle hole in the base end portion side of the first position, a biasing member that biases the slide member from the second position side to the first position side; and a cap removably mounted to the base portion and is configured to cover the needle member.

## Description

### Technical Field

The present invention relates to a needle unit configured to be attached to a medical syringe.

### Background Art

Conventionally, health hazards to healthcare workers who handle cytotoxic drugs used for cancer chemotherapy and the like have been considered a problem. The reason is that cytotoxic drugs are usually distributed in a state of being encapsulated in a drug container, and a healthcare worker has to pierce the cover (portion) of the container with a needle set in a syringe, and dissolve, dilute, mix, or extract the drug in the container. During such work, especially after removing the needle from the cover (portion), the drug remaining in the needle hole often drips out and thus the healthcare worker is subject to significant exposure to the drug.

To solve the problem, for example, Patent Literature 1 discloses a device that prevents a needle from being exposed to the outside by covering the whole needle when removing the needle from the cover portion of the drug container.

Also, Patent Literature 2 discloses a syringe for blood collection in which a needle cover covering the needle is attached to a base portion of the needle in order to prevent the leakage of blood. This needle cover remains attached to the base portion of the needle even while the needle is plaeced in a vein. That is, the needle is inserted into a vein vessel and blood collection is conducted by piercing and penetrating the needle cover from inside with the needle. Then, when the needle is withdrawn from the blood vessel after the blood drawing process is finished, the needle cover automatically returns to the original shape due to the elasticity of the needle cover, and the needle is housed in the needle cover again. Also, a through-hole on the needle cover formed by the passage of the needle is also automatially closed, due to the elasticity of the needle cover, and thus, the scattering of blood is prevented. This needle cover can be used for not only blood drawing, but also for extracting drugs which may be a problem when exposed to the outside, such as drugs with cytotoxicity, odor, and irritant properties, from the drug containers such as a vial.

Furthermore, Patent Literature 3 discloses a needle unit covering the needle tip with a cover biased by a spring or the like, in order to prevent the needle tip from being accidentally touched or a finger being pricked with the needle tip.

### Citation List

### Patent Literature

Patent Literature 1: JP 60-501294A
Patent Literature 2: JP-U 48-81693
Patent Literature 3: JP-T 3-504205A

### Summary of Invention

### Technical Problem

However, the device according to Patent Literature 1 has a problem in that applicable drug containers are limited, because the member covering the whole needle is fixed to the cover portion of the drug container. Accordingly, when pouring the collected drug into another infusion container or the like, it is necessary to take measures such as attaching a dedicated attachment to the opening of the container. In addition, since a removal operation to release the lock of the device is also needed, it takes time and effort and is bothersome for healthcare workers. In addition, since the structure of the device is complicated, there is also a problem in that the whole device is bulky and is, moreover, costly to produce.

Also, with a syringe whose needle is covered by a needle cover as in Patent Literature 2, the needle is covered again by the needle cover being restored to the original shape due to the elasticity of the needle cover, although there is a risk that a slight timelag will occur in the restoring depending on the amount of frictional force against the needle. Thus, the effect of preventing leakage of the drug is not satisfactory.

On the other hand, with regard to the needle unit of Patent Literature 3, although accidental pricking can be prevented by covering the needle tip, preventing leakage of the drug that remains inside the needle hole is not considered at all.

The present invention has been made to solve the abovementioned problems, and an object thereof is to provide a needle unit capable of prevent a drug from leaking to the outside from a needle hole.

### Solution to Problem

The present invention provides a needle unit configured to be attached to a medical syringe, the needle unit including a needle member having a longitudinally extending inner space, and having a needle hole formed in a side surface of a front end portion and an opening formed in a base end portion, the inner space being in communication with an outside through the needle hole and the opening, a base portion configured to support the base end portion of the needle member, and communicating between the syringe and the inner space of the needle member by being attached to the syringe, a slide member movable along an outer peripheral surface of the needle member, and capable of taking at least a first position to close the needle hole, and a second position to uncover the needle hole on the base end portion side of the first position, a biasing member configured to bias the slide member from the second position side to the first position side, and a cap removably attached to the base portion and is configured to cover the needle member.

According to this configuration, the slide member movable along the needle member between the first position to close the needle hole and the second position to uncover the needle hole is provided, and this slide member is biased by the biasing member so as to be disposed at the first position when the cap is not attached.

Then, when the needle member has been stuck into the cover portion of the drug container, the slide member is pushed to the second position side by the cover portion, the needle hole is uncovered, enabling to drug to be suctioned. On the other hand, when suctioning of the drug is completed and the needle member is widthdrawn from the cover portion, due to the pressure on the slide member by the cover portion being released, the slide member is biased by the biasing member to the first position, and closes the needle hole immediately. Thus, leakage of the suctioned drug from the needle hole can be prevented.

The abovementioned needle unit can be configured such that, when the cap is attached to the base portion, the slide member is caused to move to the second position, and the inner space of the cap is in communication with the outside.

According to this configuration, the following effects can be obtained. First, the inner pressure of the drug container gradually decreases as the drug is extracted from the drug containerer with the syringe, and when the negative pressure falls to a certain level or below, extracting the drug becomes difficult. Thus, when extracting the drug, in order not to allow the pressure inside the drug container to decrease too much, air of an amount substantially equal to the drug that is extracted is injected into the drug container from the syringe. For this reason, an operation to take in the equivalent amount of the air to the inner space of the syringe is performed in advance. In order to maintain safety and sanitation, this operation is often performed in the state where the cap is attached to the needle, before attaching the injection needle to the syringe and inserting the needle into the drug container.

However, in the case of a syringe in which the needle is covered by a needle cover as in Patent Literature 2, it is not easy to take in air to the inner space of the syringe in advance, before inserting the needle to the drug container, due to the needle being housed inside the needle cover. This is because, when the air in the needle cover is suctioned into the inner space of the syringe, the needle cover, and thus further suctioning of air becomes difficult.

Therefore, according to the above configuration, the slide member moves to the second position when the cap is attached, and thus the outside of the cap, the inner space of the cap, and the needle hole are in communication with one another. As a result, suctioning of the air into the syringe with the needle unit attached thereto becomes possible. For this reason, it is possible to prevent the pressure inside the drug container from decreasing by injecting air into the drug container when the drug is suctioned from the drug container.

In the above needle unit, the biasing member can be configured in various ways. For example, the biasing member can be constituted by a coiled spring that joins the base portion and the slide member and extends along the outer peripheral surface of the needle member.

Alternatively, the biasing member can be constituted by at least one plate spring that joins the base portion and the slide member.

In particular, when a pair of the plate springs are provided, the pair of plate springs may be arranged symmetrically around the axis of the needle member.

In the above needle units, the slide member can be configured to cover the front end of the needle unit in a state where a load is not acting on the biasing member.

In this case, the slide member can be formed of a transparent or semi-transparent material. Thereby, even if the slide member covers the front end of the needle member, the front end of the needle member can be seen from the outside of the slide member.

In the above needle unit, in order to move the slide member to the second position side, the cap can be configured in various way. For example, an engaged portion configured to engage with the slide member and move the slide member to the second portion when the cap is attached to the base portion can be provided, in the inner space of the cap.

As the configuration of the engaging portion, for example, a configuration can be adopted in which the inner space includes a small diameter portion arranged on the front end side of the needle member, and a large diameter portion arranged continuously with the small diameter portion on the base end portion side, and a stepped portion between the small diameter portion and the large diameter portion constitutes the engaging portion.

Also, in order to move the slide member to the second position side, the following configuration can be adopted. That is, a configuration can be adopted in which the slide member may be provided with an extention member that covers the side surface of the front end portion of the needle member when the slide member is at the first position, and the extension member is pushed to the base portion side by the cap when the cap is attached to the base portion.

In the above needle units, the distance between the slide member and the needle member may be larger than 0 and less or equal to 50 µm. This is because if the distance between the slide member and the side surface of the needle member is 0, it becomes difficult for the slide member tends to move immediately along the needle member due to the frictional force, and if the distance is larger than 50 µm, there is a risk of leakage of the drug.

Also, in the above needle units, the needle hole can be formed at a position which is 3 mm to 10 mm from the front end of the needle member. This is because if the needle hole is positioned closer to the front end side of the needle member than the above distance, the needle hole will be formed on the inclined surface of the cornial part of the pointed front end portion of the needle member, thus will be incompletely closed by the slide member; on the other hand, if the distance from the front end exceeds 10 mm, there is a risk that the needle hole will be an obstacle to collecting the drug.

### Advantageous Effects of Invention

According to the needle unit of the present invention, it is possible to suction air into the inner space of the syringe in the state where the needle is attached to the syringe and also covered by the cap, while preventing leakage of the drug to the outside from the needle hole.

### Brief Description of Drawings

FIG. 1 is a cross-sectional view of a syringe to which a needle unit according to an embodiment of the present invention is attached;
FIG. 2 is a perspective view showing the needle unit according to an embodiment of the present invention in the state where a cap is attached thereto;
FIG. 3 is a perspective view showing the needle unit in FIG. 2 in the state where the cap is removed therefrom;
FIG. 4 is a cross-sectional view showing the needle unit in FIG.3 in the state where the cap is removed therefrom;
FIG. 5 is a cross sectional view of FIG. 2;
FIG. 6 is a side cross sectional view of a drug container;
FIG. 7 is a diagram illustrating the method for using the needle unit in FIG. 2;
FIG. 8 is a diagram illustrating the method for using the needle unit in FIG. 2;
FIG. 9 is a diagram illustrating the method for using the needle unit in FIG. 2;
FIG. 10 is a diagram illustrating the method for using the needle unit in FIG. 2;
FIG. 11 is a cross-sectional view showing another example of the needle unit in FIG. 2, and showing the state where the cap is removed;
FIG. 12 is a cross-sectional view showing the needle unit in FIG. 11 in the state where a cap is attached;
FIG. 13 is a cross-sectional view showing another example of the needle unit according to the present invention, showing the state where the cap is removed;
FIG. 14 is a cross-sectional view showing another example of the needle unit according to the present invention, showing the state where the cap is attached; and
FIG. 15 is a cross-sectional view showing another example of the needle unit according to the present invention, showing the state where the cap is attached.

### Description of Embodiments

In the following, an embodiment of the needle unit according to the present invention will be described with reference to the drawings. A needle unit 10 according to this embodiment is to be attached to a medical syringe 20. In the following, first, the medical syringe 20, then, the needle unit 10 will be described, and after that, the method for using the needle unit 10 will be described.

### 1. Syringe

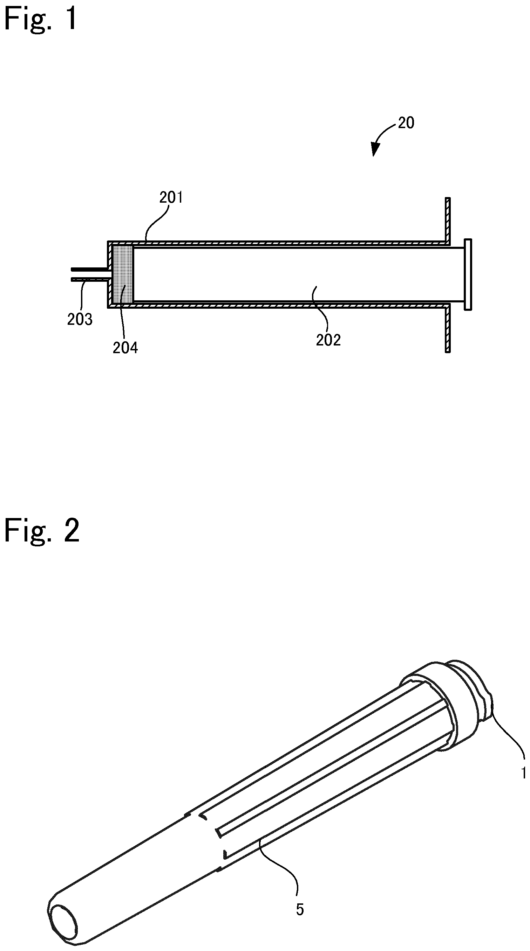

Fig. 1 is an example of a syringe 20 according to this embodiment. This syringe 20 is well known, and provided with a hollow external cylinder 201, and a plunger 202 inserted from the opening at the rear end of the external cylinder 201. At the front end of the external cylinder 201, a nozzle 203 with a small diameter is mounted, and the needle unit 10 is attached to this nozzle 203. Also, at the front end of the plunger 202 inserted to the external cylinder 201, a gasket 204 is mounted, thereby sealing the rear end of the inner space of the external cylinder 201.

### 2. Needle Unit

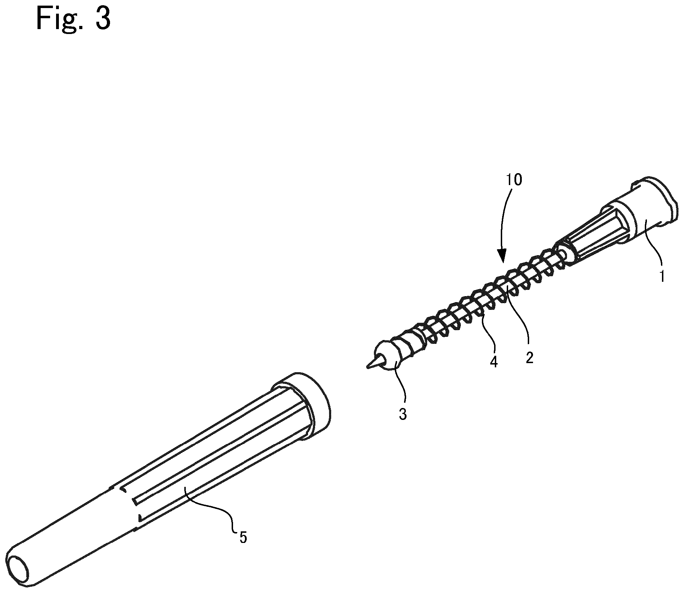

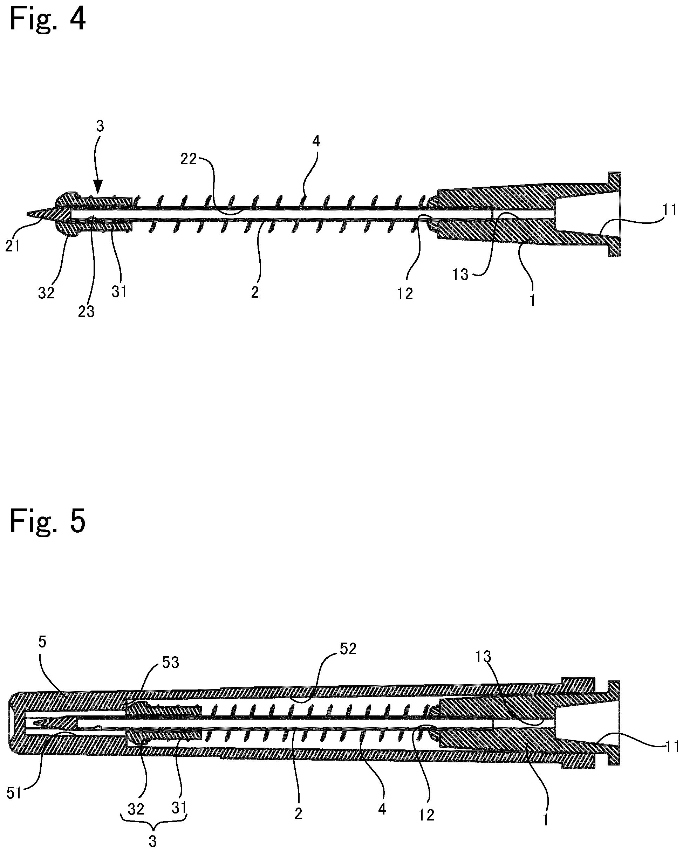

Next, the needle unit will be described. FIG.2 is a perspective view showing the needle unit in the state where a cap is attached, FIG. 3 is a perspective view showing the needle unit in the state where the cap is removed, Fig. 4 is a cross-sectional view of the needle unit in FIG. 3, FIG. 5 is a cross-sectional view of FIG. 2. Note that, in the following, the direction to which the needle member shown in FIG. 5 extends is referred to as an axis direction for convenience of description. Also, the left side in FIG. 4 is referred to as the front end side or the front side, and the right side is referred to as the base end side or the rear end side. Based on these directions, the other drawings will be described. Note that the present invention is not limited by these directions.

As shown in FIGS. 2 to 5, this needle unit 10 is provided with a base portion 1 to be attached to a nozzle 203 of the syringe 20, and a needle member 2 to be attached at the front end side of the base portion 1. This needle unit 10 is further provided with a slide member 3 attached to the needle member 2, a spring member biasing this slide member 3 to the front end side of the needle member 2, and a cap 5 covering the needle member 2. In the following, each of the members will be described in detail.

The base portion 1 is formed of rigid plastic, glass, metal, or the like, and formed in a cylindrical shape whose external diameter becomes smaller toward the front end. Then, on the base end side of the base portion 1, a recess portion 11 is formed, into which the nozzle 203 of the syringe 20 is inserted. On the other hand, at the front end of the base portion 1, an insertion slot 12 is formed, into which the needle member 2 is inserted, and this insertion slot 12 and the front end of the recess portion 11 are in communication with each other via a communication path 13. Note that, although the nozzle 203 of the syringe 20 and the base end portion 1 are fixed by inserting the nozzle 203 into the recess portion 11, there is no limitation in particular to the method for fixing them. For example, so-called lure lock fixing is also possible as well as a fixing simply by press-fit. In the lure lock fixing, the nozzle 203 and the base portion 1 are fixed by providing a wall with a female screw around the nozzle 203 and screwing a male screw formed on the outer peripheral surface of the base portion 1 into the female screw.

Next, the needle member 2 will be described. The needle member 2 according to this embodiment is a well known needle member that is also called a bud needle and is formed of rigid metal, plastic, or the like. More specifically, this needle member 2 has a front end portion 21 that is formed in a pointed circular cone, and an inner space 22 extending in the longitudinal direction. Then, this inner space 22 has an opening on the base end side, and is in communication with the insert slot 12 of the base portion 1. Also, on the side surface near the front end of the needle member 2 (on the base end side of the circular cone part), a needle hole 23 is formed, and the needle hole 23 is in communication with the inner space 22. Accordingly, the recess portion 11 of the base portion 1, the communication path 13, the inner space 22 of the needle member 2, and the needle hole 23 are in communication with one another, and the drug that flows in from the needle hole 23 can flow in the syringe 20 via the base portion 1.

Note that, it is preferable that the needle hole 23 is formed at a position that is 3 to 10 mm from the front end of the needle member 2, that is, on the base portion 1 side of the inclined surface of the circular cone part of the front end portion 21. This is because, if the needle hole 23 is at a position which is closer to the front end than that, the needle hole 23 will be formed on the inclined surface of the circular cone of the front end portion 21. Therefore, as mentioned later, the slide member 3 cannot close the needle hole completely. On the other hand, if the distance from the front end exceeds 10mm, there is a risk that the needle hole will be an obstacle to collecting the drug. Also, it is preferable that the needle hole 23 is formed in a circle or an oval, whose diameter is 0.5 to 1.2mm, depending on the outer diameter of the needle member 2. In addition, it is also possible to provide a plurality of the needle holes 23. In this case, it is also possible to arrange the needle holes 23, symmetrically around the axis of the needle member 2, as well as in a row along the axis direction. However, if there are too many needle holes 23, the needle holes cannot be closed by the slide member 3, and thus, the number of the needle holes should be at least within a number which can be covered with the slide member 3.

Also, a cylindrical slide member 3 is attached to the needle member 2, and the slide member 3 is movable in the axis direction along the needle member 2. This slide member 3 is provided with a cylindrical main body 31, and a cylindrical engaging portion 32 to be attached at the front end side of the main body 31 with a larger diameter than that of the main body 31, and into a through-hole penetrating the main body 31 and the engaging portion 32, the needle member 2 is inserted. Also, between the rear end portion of the slide member 3 and the front-end portion of the base portion 1, a coiled spring member (biasing member) 4 is attached. In other words, the needle member 2 is inserted into this spring member 4, the front end of the spring member 4 is fixed to the rear end portion of the slide member 3, and the rear end portion of the spring member 4 is fixed to the front end portion of the base portion 1. As shown in FIG. 4, the slide member 3 is biased to the front end side of the needle member 2 by this spring member 4, and when the spring member 4 is not compressed, the slide member 3 is positioned near the front end of the needle member 2 (a first position), and the slide member 3 covers the needle hole 23. Then, as described later, when the cap 5 is attached so as to cover the needle member 2, the slide member 3 is biased to the base portion 1 side by the cap 5, thereby compressing the spring member 4. As a result, the slide member 3 moves to the base portion 1 side (a second position) so as to expose the needle hole 23.

Note that, thermoplastic elastomer, polyethylene, polypropylene, poly-4-methylpentene, or polycarbonate can be used as the slide member 3, and in addition, resin with a small frictional force, such as polytetrafluoroethylene, silicone resin, or the like, can be also used preferably. In addition, if necessary, silicone coating or silicone application is also possible. It is also preferable that the gap between the inner wall surface of the through-hole of the slide member 3 and the side surface of the needle member 2 is larger than 0mm, and less than or equal to 50 µm. This is because when the gap between the two is 0, it is difficult for the slide member 3 to move immediately along the needle member 2, and when the gap is larger than 50 µm, as described later, there is a risk of a leakage of the drug from the needle hole 23 even if the slide member 3 covers the needle hole 23.

Next, the cap 5 will be described. The cap 5 is formed in a hollow cylinder configured to cover the needle member 2, and has a closed front end and an open rear end. Then, the opening of the rear end portion is configured to be engaged with the front end portion of the base portion 1. Additionally, on the inner wall surface of the cap 5, a small diameter portion 51 is formed near the front end portion in the axis direction, and on the rear end side of the small diameter portion 51, a large diameter portion 52 with a large diameter is formed. Thus, a stepped portion 53 is formed between the small diameter portion 51 and the large diameter portion 52 of the cap 5, and this stepped portion 53 is configured to be engaged with the engaging portion 32 of the slide member 3. That is, the internal diameter of the small diameter portion 51 of the cap 5 is smaller than the external diameter of the engaging portion 32 of the slide member 3, and the internal diameter of the large diameter portion 52 is larger than the external diameter of the engaging portion 32. Therefore, as shown in FIG. 5, in the state where the cap 5 is attached to the base portion 1, the stepped portion 53 is configured to engage with the engaging portion 32 of the slide member 3, and push the engaging portion 32 to the base portion 1 side. That is, the slide member 3 is configured to move from the position to cover the needle hole 23 so as to expose the needle hole 23.

Furthermore, when the cap 5 is attached to the base portion 1, a slight gap (not shown) is formed between the rear end portion of the cap 5 and the base portion 1, whereby the inner space of the cap is in communication with the outside.

Note that it is preferable that the cap 5 can be formed of, for example, a general-purpose rigid plastic such as polyethylene, polypropylene, or the like, and that the cap 5 is transparent or semi-transparent so that the inside can be seen.

### 3. Method for Using Needle Unit

In the following, the method for collecting the drug housed in the drug container 30 with the needle unit 10 attached to the syringe 20 will be described. First, prior to the description of the method of usage, an example of the drug container used here will be described.

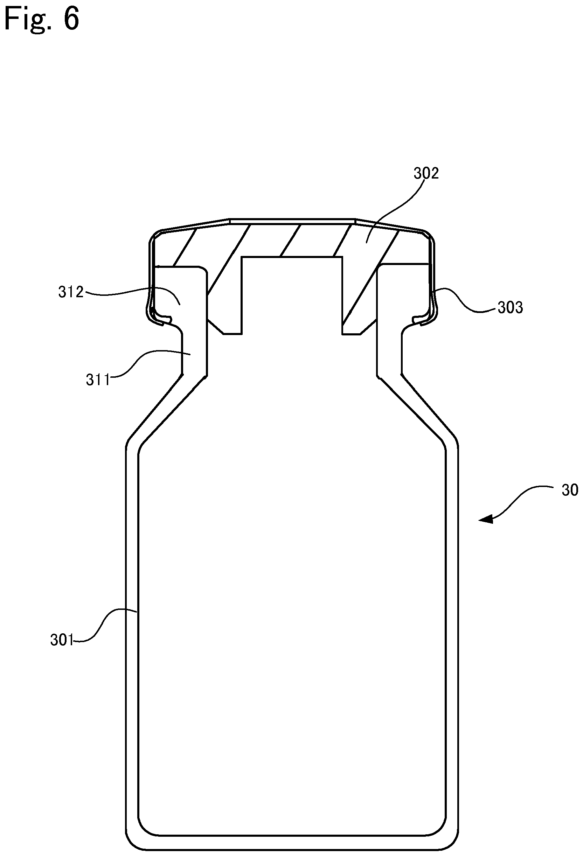

As shown in the FIG. 6, this drug container 30 is generally called a vial container, which is provided with a bottle body 301 formed of glass, and a bottle cap (cap portion) 302 to close an opening formed at the top of the bottle body 301. The bottle body 301 is typically transparent or semi-transparent. The bottle body 301 is substantially cylindrical as a whole, and a neck portion 311 with a small diameter is formed on the upside. Furthermore, a flange portion 312 is formed on the upside of the neck portion 311, and the bottle cap 302 is attached so as to cover this flange portion 312.

The part of the bottle cap 302 which closes the upper opening of the bottle body 301 is formed of a material which is capable of elastic deformation such as rubber, elastomer, or the like, and as described later, capable of being pierced with the needle member 2. On the contrary, when the needle member 2 is drawn out of this part, the pinhole formed by the piercing of the needle member 2 is almost instantly closed due to its elasticity, though not completely. Furthermore, the bottle cap 302 is seam-fixed to the flange portion 312 with an aluminum cap 303, and this aluminum cap 303 covers the whole part of the bottle cap 302 except the central part of the top surface. Accordingly, when accessing inside the drug container 30 by using the syringe 20, it is necessary to pierce the needle member 2 through the circular central part on the top surface of the bottle cap 302.

There is no limitation for the drug contained in the drug container 30 in particular, and it is, for example, a drug which may be problematic when exposed to the outside. For example, such a drug is a cytotoxic drug, the exposure of which to the outside may give a serious side effect, or a health hazard due to the cytotoxicity, to those who handle the drug (mainly, healthcare workers; also referred to as a user). Examples of such drugs are anti-malignant tumor agent, immune-suppressive agent, anti-virus agent, antibiotic agent, radioactive agent, and the like. Also, other examples of the drug which may be problematic when exposed to the outside are drugs with odor, irritant property, and the like. Note that the drugs include powdered drug or the like, as well as liquid drug. When suctioning a powdered drug, a mixing liquid is filled inside the drug container 30 by using the needle unit 10 and the syringe 20 prior to the suctioning, then the drug is dissolved by a mixing liquid in the drug container 30. The mixing liquid is, for example, normal saline, Ringer's solution, distilled water, and the like, which is a solution for diluting or dissolving the drug.

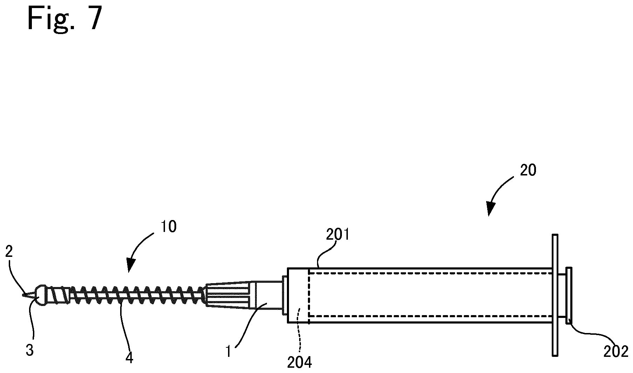

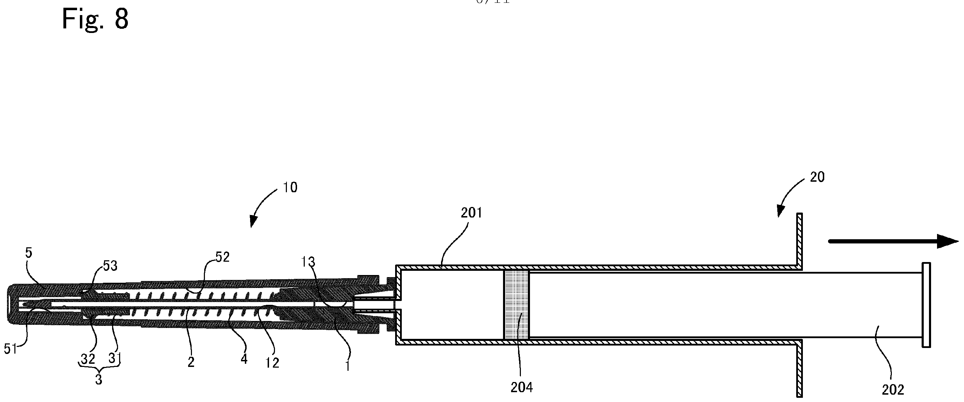

Next, the method for using the needle unit 10 will be described. First, as shown in FIG. 7, the base portion 1 of the needle unit 10 is fixed to the nozzle 203 of the syringe 20. At this time, the plunger 202 of the syringe 20 remains pressed into the external cylinder 201, that is, the gasket 204 remains pressed against the front end portion of the external cylinder 201. Then, as shown in the FIG. 8, the cap 5 is attached to the base portion 1, and the plunger 202 is moved backward to suction air into the external cylinder 201. At this time, a gap is formed between the opening in the base end portion of the cap 5 and the base portion 1, the slide member 3 is at the second position to uncover the needle hole 23, and the needle hole 23 is exposed. Whereby, when the plunger 202 is moved backward, the air flowing from the gap and the needle hole 23 is suctioned into the external cylinder 201.

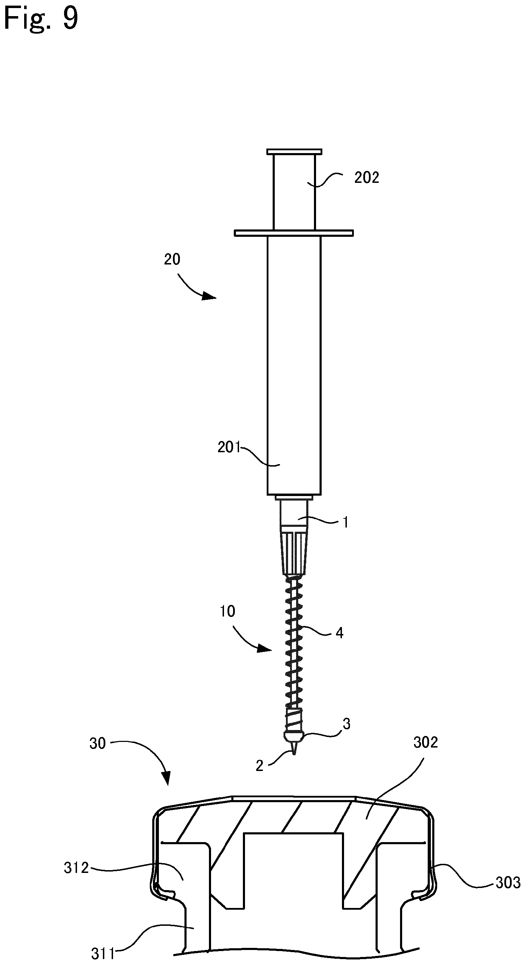

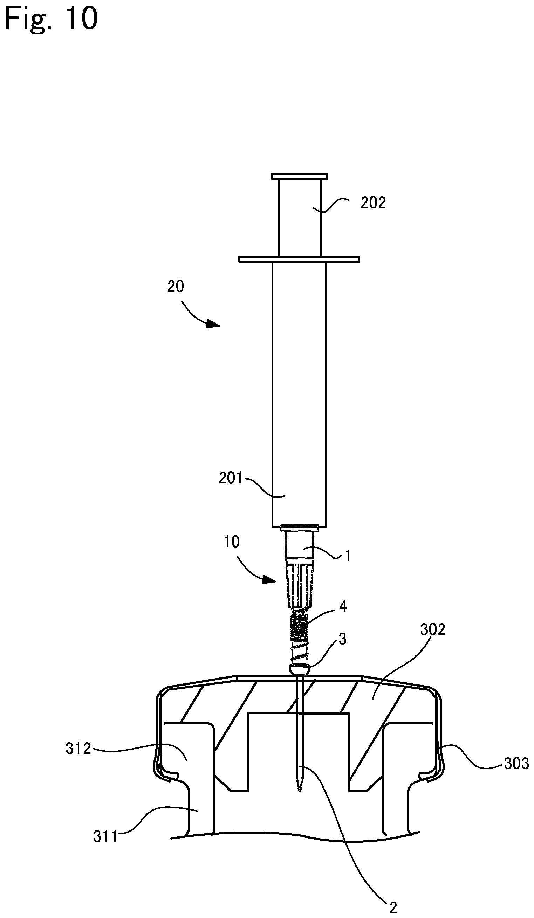

Next, the cap 5 is removed from the base portion 1. Thus, due to a load pressing the slide member 3 to the base portion 1 side being released, as shown in FIG. 9, the slide member 3 is biased to the front side by the spring member 4 and is positioned to cover the needle hole 23. In this state, the needle member 2 is pierced into the bottle cap 302 of the drug container 30, and the drug is suctioned while sending the air into the drug container 30 by a pumping operation. At this time, as shown in FIG. 10, when the needle member 2 is pierced into the bottle cap 302, the slide member 3 is pressed by the bottle cap 302 and moved to the base portion 1 side. That is, due the slide member 3 being movable along the needle member 2, the slide member 3 is not an obstacle for the needle member 2 to pierce the bottle cap 302.

Then, when a desired amount of the drug has been collected to the external cylinder 201 of the syringe 20, the needle member 2 is drawn out of the bottle cap 302. At this time, as shown in FIG. 9, the slide member 3 which has been thrusted by the bottle cap 302 is, due to the release of the pressure by the bottle cap 302, biased to the front end side by the spring member 4, and then positioned to cover the needle hole 23. That is, the leakage of the suctioned drug from the needle hole 23 is prevented due to the slide member 3 closing the needle hole 23 instantly at the same time the needle member 2 is drawn out of the bottle cap 302.

Then, after suctioning the drug from the drug container 30, the user pierces the needle member 2 into the bottle cap of a mixing liquid container in which the mixing liquid is contained, and thrusts the plunger 202. Thus, the drug in the syringe 20 is filled into the mixing liquid container, and the drug and the mixing liquid are mixed. Then, as required, the drug is suctioned from the plurality of drug containers 30, and filled into the mixing liquid container accordingly. In this way, a mixed medical solution is prepared. Note that, when the suctioning as described above is conducted from a plurality of drug containers 30, it is possible to use a common syringe 20, and it is also possible to change the syringe 20 partway through the suctioning. After that, the user carries the mixing liquid container containing the mixed medical liquid to a patient, and administers the mixed medical liquid in the mixing liquid container to the patient by the way of infusion or the like.

### 4. Features

As described above, according to the needle unit 10, the slide member 3 is provided with the slide member 3 that is capable of moving between the position to close the needle hole 23 and the position to uncover the needle hole 23, and the slide member 3 is biased by the spring member 4 so as to be positioned to close the needle hole 23 when the cap 5 is not attached. On the other hand, when the cap 5 is attached, while the slide member 3 is moved to the position to uncover the needle hole 23, the inner space of the cap 5 becomes in communication with the outside.

Thus, when the cap 5 is attached, the outside of cap 5, the inner space of the cap 5, and the needle hole 23 are in communication with each other, thus the air can be suctioned into the syringe 20 with the needle hole 10 is attached thereto. For this reason, it is possible to prevent the decrease of the pressure inside the drug container 30 by filling air into the drug container 30 at the time when the drug is suctioned from the drug container 30.

Then, when the needle member 2 is pierced into the bottle cap 302 of the drug container 30, due to the bottle cap 302 pressing the slide member 3 to the base portion 1 side, the needle hole 23 is uncovered, and then the drug can be suctioned. On the other hand, when the suction of the drug is completed and the needle member 2 is drawn out of the bottle cap 302, due to the pressure on the slide member 3 by the bottle cap 302 being released, the slide member 3 is biased to the front side by the spring member 4, and covers the needle hole 23 instantly. Thus, leakage of the suctioned drug from the needle hole 23 can be prevented.

### 5. Modified Examples

As above, some embodiments of the present invention have been described, however the present invention is not limited to the above embodiments, and various changes are possible without departing from the spirit of the invention. In addition, the gist of the other embodiments as described in the following can be combined if necessary.

### <1>

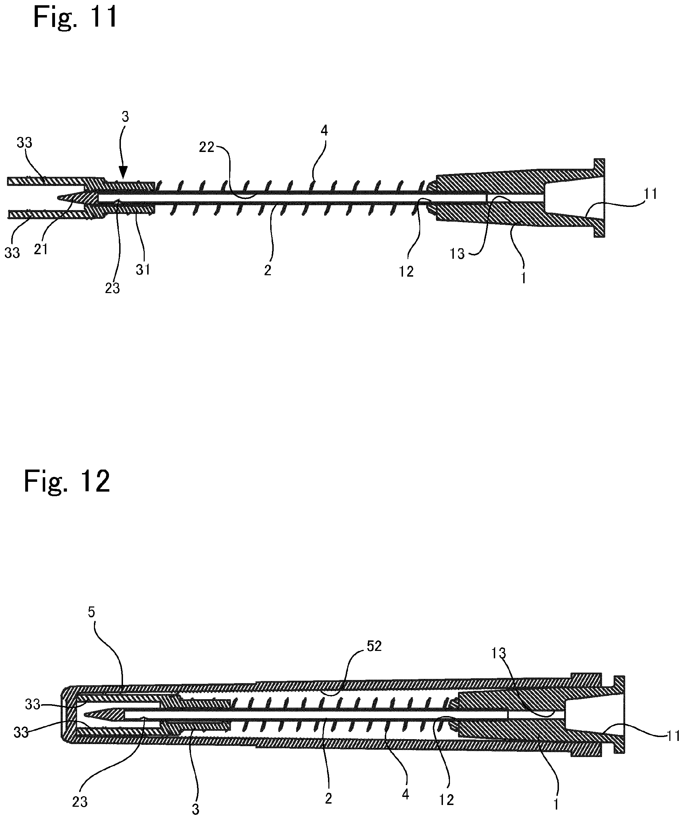

In the above embodiment, the stepped portion 53 is provided to the inner wall surface of the cap 5 so as to press the slide member 3 to the base portion 1 side when the cap 5 is attached to the base portion 1. However, other configurations are also possible. For example, it is also possible to provide a protrusion portion (engaged portion) to the inner surface of the cap 5, engage this protrusion part with the engaging part 32 of the slide member 3, and thus move the slide member 3 to the base portion 1 side. Alternately, as shown in FIG. 11, a pair of extension members 33 extending forward is attached to the front end portion of the slide member 3. Each of the extension members 33 is formed in a sectional circular arc, and the two extension members 33 are configured to surround the front end portion 21 of the needle member 2 from the sides. Thus, when the slide member 3 closes the needle hole 23, the front end portion 21 of the needle member 2 can be protected by the extension members 33.

On the other hand, when the cap 5 is attached to the base portion 1, as shown in FIG. 12, the front end surface of the cap 5 presses both extension members 33, thus the slide member 3 moves to the base portion 1 side. As a result, the needle hole 23 is uncovered. Note that, there is no limitation in particular to the configuration of the extension member 33, as long as it is formed to be pressed by the front end portion of the cap 5, without using a stepped part. In addition, it is also possible to use one extension member, or three or more extension members.

In this way, there are many ways to move the slide member 3 when the cap 5 is attached to the base portion 1, which are not particularly limited.

### <2>

In the above embodiment, the gap is formed between the rear end of the cap 5 and the base portion 1 such that air can flow into the inner space of the cap 5. However, other configurations are also possible as long as air can flow in the inner space of the cap 5. For example, it is also possible to form the penetrating hole on the side surface of the cap 5 to make air flow thereinto.

### <3>

In the above embodiment, for example, as shown in FIGS. 3 and 4, the front end portion 21 of the needle member 2 slightly protrudes from the front end of the slide member 3 such that the position of the front end portion 21 of the needle member 2 is visible. A configuration is also possible to adjust the position of the slide member 3 so as not to protrude from the slide member 3 and to house contain the front end portion 21 of the needle member 2 in the slide member 3. In this case, for example, forming the slide member 3 of a transparent or semi-transparent material allows the position of the needle member 2 to be visible even if the front end portion 21 of the needle member 2 is covered with the slide member 3.

### <4>

There is no limitation in particular to the configuration of the slide member 3, as long as that it is movable along the needle member 2 and formed capable of closing the needle hole 23. Also, there is no limitation in particular to the second position according to the present invention, as long as it is a position to uncover the needle hole 23. Similarly, there is no limitation in particular to the configuration of the base portion 1, as long as it supports the needle member 2 and is capable of being attached to the syringe 20.

### <5>

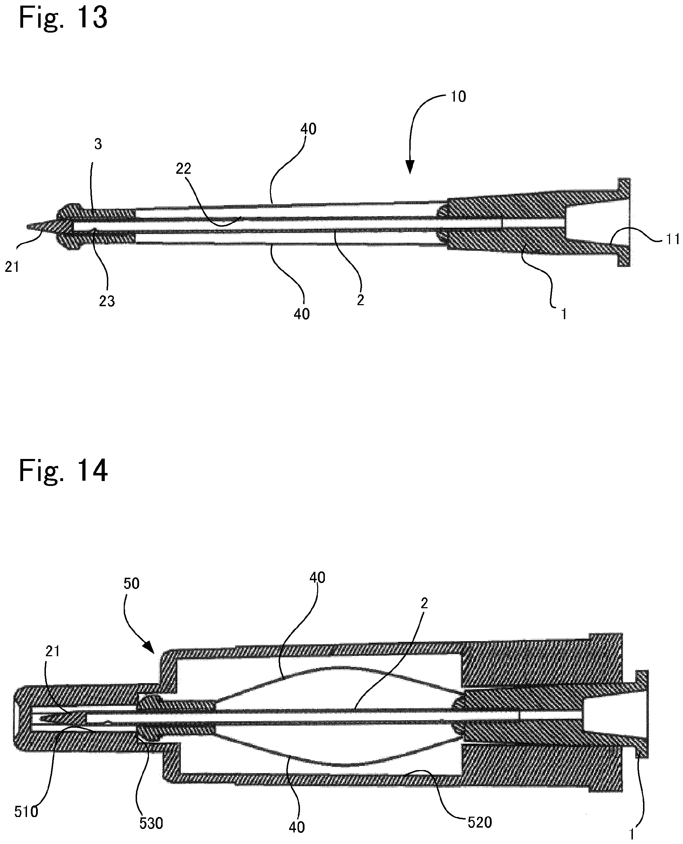

Furthermore, there is no limitation in particular to the biasing member of the present invention, as long as the biasing member is capable of biasing the slide member 3 from the position on the base portion 1 side to the position to close the needle hole 23, like the spring member in the abovementioned embodiment. For example, a plate spring can be used as the spring member. Specifically, as shown in FIG. 13, a pair of spring members 40 formed of plate springs are provided, and the slide member 3 and the base portion 1 are linked by them. At this time, both spring members 40 are positioned symmetrically around the axis of the needle member 2. As also shown in the figure, when the cap 50 is not attached to the needle unit 10, both spring members 40 extends substantially linearly without bending.

Also, as shown in FIG. 14, the cap 50 is formed to be capable of housing the bent spring member 40. Specifically, like the above embodiment, the cap 50 according to this embodiment is also provided with a small diameter portion 510 containing the front end portion 21 of the needle member 2 and a large diameter portion 520 that is closer to the base portion 1 than a small diameter portion 510, and a stepped portion 530 is formed between them. Thus, when the cap 50 is attached to the needle unit 10, the slide member 3 is pressed to the base portion 1 side by the stepping portion 530. At this time, each of the spring members 40 bends such that the needle member 2 is separated therefrom, and the large diameter portion 520 of the cap 50 is formed in a rectangle that can contain both bent spring members 40.

The configuration of the spring member 40 by the plate springs as described above has the following advantages. For example, if the spring member is coiled as in the above embodiment, there is a risk that the slide member 3 will fly out of the front end of the needle member 2 by the rebound of the compressed spring member 4 when the needle member as shown in FIG. 10 is removed after piercing into the bottle cap 302 of the drug container 30. On the other hand, if the spring member 40 is configured of a plate spring, and arranged at the position such that the slide member 3 covers the needle hole 23 with the spring plate 40 extending substantially linearly when the cap 50 is not attached to the needle unit 10, the movement of slide member 3 from this position to the front end side is restricted. Accordingly, it is possible to prevent the slide member 3 from dropping off from the needle member 2 even if the spring member 40 reacts from the state where the slide member 3 is moved to the base portion 1 side and the spring member 40 is bent.

Note that, in the above embodiment, the spring member 40 and the slide member 3 can be formed of different materials, or formed of a material in one piece. In addition, it is also possible to provide one spring member 40, or three or more spring members, as well as the two spring members.

Furthermore, in the case where the coiled spring member 4 is provided, for example, it is also possible to link the slide member 3 and the base portion 1 with a deformable restricting member which is shaped like the above plate spring. In this way, even if the coiled spring member 4 is used, it is possible to prevent the slide member 3 from dropping off from the needle member 2 by the restricting member. Note that, there is no limitation in particular to the material configuring the spring member 40 by the plate spring, as long as it is formed of an elastic material with resilience that performs spring function, such as metal, plastic, or the like.

### <6>

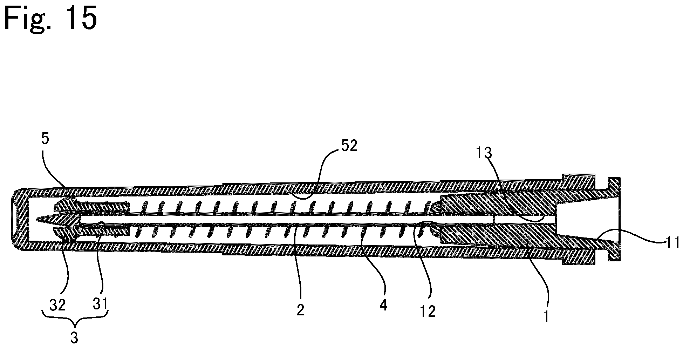

In the above embodiment, although it is configured that the slide member 3 is pressed to the base portion 1 side by the cap 5 when the cap 5 is attached so as to uncover the needle hole 23, this is not essential. For example, as shown in FIG. 15, it is also possible not to provide the small diameter portion 51 and the stepped portion 53 at the cap 51 such that the small diameter portion 3 does not move when the cap 5 is attached to the base portion 1. Even in this way, if it is configured such that a slight gap is formed between the slide member 3 and the needle hole 23, and the inside and the outside of the cap 5 are in communication with each other, it is possible to let air flow in from the needle hole 23 by pulling the plunger 202. Of course, in a hygienic condition, it is also possible to let air flow into the inner space of the syringe 20 before attaching the needle unit 10 to the nozzle 203 of the syringe 20.

### List of Reference Numerals

- 1: base portion
- 2: needle member
- 23: needle hole
- 3: slide member
- 33: extension member
- 4: spring member (biasing member)
- 40: spring member (biasing member)
- 5: cap
- 50: cap
- 10: needle unit
- 20: syringe
- 51: small diameter portion
- 52: large diameter portion
- 53: stepped portion (engaged portion)
- 510: small diameter portion
- 520: large diameter portion
- 530: stepped portion (engaged portion)

## Claims

1. A needle unit configured to be attached to a medical syringe, comprising:
a needle member having a longitudinally extending inner space, and having a needle hole formed in a side surface of a front end portion and an opening formed in a base end portion, the inner space being in communication with an outside through the needle hole and the opening;
a base portion configured to support the base end portion of the needle member, and communicating between the syringe and the inner space of the needle member by being attached to the syringe;
a slide member movable along an outer peripheral surface of the needle member, and capable of taking at least a first position to close the needle hole, and a second position to uncover the needle hole in the base end portion side of the first position;
a biasing member configured to bias the slide member from the second position side to the first position side; and
a cap removably attached to the base portion and configured to cover the needle member.

2. The needle unit according to Claim 1, wherein
the needle unit is configured such that, when the cap is attached to the base portion, the slide member is caused to move to the second position, and the inner space of the cap is in communication with the outside.

3. The needle unit according to Claim 1 or 2, wherein
the biasing member is constituted by a coiled spring that joins the base portion and the slide member and extends along the outer peripheral surface of the needle member.

4. The needle unit according to Claim 1 or 2, wherein
the biasing member is constituted by at least a plate spring that joins the base portion and the slide member.

5. The needle unit according to Claim 4, wherein
a pair of the plate springs are provided, and
the pair of plate springs are arranged symmetrically around the axis of the needle member.

6. The needle unit according to any one of Claims 1 to 5, wherein
the slide member is configured to cover the front end of the needle unit in a state where a load is not acting on the biasing member.

7. The needle unit according to Claim 6, wherein
the slide member is formed of a transparent or semi-transparent material.

8. The needle unit according to any one of Claims 2 to 7, comprising:
an engaged portion configured to engage with the slide member and move the slide member to the second portion when the cap is attached to the base portion, in the inner space of the cap.

9. The needle unit according to Claim 8, wherein
the inner space comprises:
a small diameter portion arranged on the front end side of the needle member, and
a large diameter portion arranged continuously with the small diameter portion on the base end portion side, and
a stepped portion between the small diameter portion and the large diameter portion constitutes the engaged portion.

10. The needle unit according to any one of Claims 1 to 9, wherein
the slide member comprises:
an extension member configured to cover the side surface of the front end portion of the needle member, when the slide member is at the first position, and
the extension member is configured to be pushed to the base end portion side by the cap, when the cap is attached to the base portion.

11. The needle unit according to any one of Claims 1 to 10, wherein
a distance between the slide member and the side surface of the needle member is larger than 0 µm and less than or equal to 50 µm.

12. The needle unit according to any one of Claims 1 to 11, wherein
the needle hole is formed at a position which is 3 to 10 mm from the front end of the needle member.
